(19) Europäisches Patentamt
European Patent Office
Office européen des brevets

(11) Veröffentlichungsnummer: **0 026 154**
**B1**

# (12) EUROPÄISCHE PATENTSCHRIFT

(45) Veröffentlichungstag der Patentschrift: **29.06.83**

(51) Int. Cl.³: **C 07 C 143/55,** C 07 C 143/62, C 09 B 23/14

(21) Anmeldenummer: **80810183.6**

(22) Anmeldetag: **02.06.80**

(54) **Verfahren zur Herstellung von 4,4'-Dinitrostilben-2,2'-disulfonsäure und deren Salzen und deren Verwendung.**

(30) Priorität: **08.06.79 CH 5369/79**

(43) Veröffentlichungstag der Anmeldung: **01.04.81 Patentblatt 81/13**

(45) Bekanntmachung des Hinweises auf die Patenterteilung: **29.06.83 Patentblatt 83/26**

(84) Benannte Vertragsstaaten: **AT BE CH DE FR GB IT LI NL**

(56) Entgegenhaltungen:
**DE-A-2 258 530**

**JOURNAL OF ORGANIC CHEMISTRY, Band 32, Januar 1967, Baltimore G.A. RUSSELL et al. "Oxidation of Carbanions. II. Oxidation of p-Nitrotoluene and Derivatives in Basic Solution" Seiten 137 bis 146**

***Die Akte enthält technische Angaben, die nach dem Eingang der Anmeldung eingereicht wurden und die nicht in dieser Patentschrift enthalten sind.***

(73) Patentinhaber: **CIBA-GEIGY AG, Patentabteilung Postfach, CH-4002 Basel (CH)**

(72) Erfinder: **Guglielmetti, Leonardo, Dr., Wartenbergstrasse 6, CH-4103 Bottmingen (CH)**

**EP 0 026 154 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 4,4′-Dinitrostilben-2,2′-disulfonsäure und deren Salzen und deren Verwendung

Die vorliegende Erfindung betrifft ein Verfahren zur Herstellung von 4,4′-Dinitrostilben-2,2′-disulfonsäure und deren Salzen und deren Verwendung.

Die Verfahren zur industriellen Herstellung der 4,4′-Dinitrostilben-2,2′-disulfonsäure und deren Salze sind allgemein bekannt und bestehen in der oxydativen Kondensation von 2 Mol 4-Nitrotoluol-2-sulfonsäure unter wässrigen alkalischen Bedingungen. Als Oxydationsmittel wurden Sauerstoff (Luft) in Gegenwart eines Katalysators oder Natriumhypochlorit beschrieben (vgl. z.B. A.G. Green und A.R. Wahl, B. 30, 3097–3101 (1897); 31, 1079 (1898); DRP 106.961; C. 1900 I, 1085; DRP 113.514 und C. 1900 II, 703). Diese Verfahren liefern jedoch die 4,4′-Dinitrostilben-2,2′-disulfonsäure und deren Salze, trotz modernen technischen Verbesserungen, nur in verhältnismässig schlechten Ausbeuten, die zwischen 60 und 75% liegen (vgl. z.B. DE-OS 2 258 530).

In den letzten 15 Jahren wurden zahlreiche Anstrengungen unternommen, um die Ausbeute dieser Kondensation unter Anwendung physikochemischer, mathematischer und analytischer Methoden sowie von Computer-Modellen zu verbessern. Diese Anstrengungen blieben aber erfolglos [vgl. z.B. C.A. 83, 113.377 h (1975); C.A. 85, 192.288 z, 192.289 a, 192.290 n (1976); C.A. 86, 16029c (1977); Chimie Analytique 50, 251–254 (1968) und Chimie et Industrie, Genie Chimique 101, 1439–1447 (1969)].

Die Verwendung von wässrigen Natriumhypochlorit-Lösungen als Oxydationsmittel bringt zahlreiche Nachteile mit sich. Die Oxydation verläuft sehr schnell und muss auch sehr schnell durchgeführt werden, damit die Ausbeute durch Bildung von gefärbten Nebenprodukten nicht nachteilig beeinflusst wird. Da die Oxydation aber auch stark exotherm verläuft, müssen auch Wärmeabfuhrprobleme gelöst werden. Es müssen ausserdem grosse Flüssigkeitsmengen transportiert, verarbeitet und gelagert werden, deren Aktivität sich in Abhängigkeit von der Temperatur, dem Baumaterial der Vorratsbehälter und geringen Mengen an Verunreinigungen, die in wässrigen Natriumhypochlorit-Lösungen immer vorhanden sind, ständig ändert. Diese Instabilität ist während der warmen Jahreszeit besonders ausgeprägt. Ausserdem weisen die grossen Mengen NaCl, die in den wässrigen Natriumhypochlorit-Lösungen ständig vorhanden sind, ökologisch und wirtschaftlich nachteilige Wirkungen auf. Die Oxydation kann somit wegen der Begrenzung der zulässigen Konzentration der während der Oxydation gebildeten Zwischen- und Nebenprodukte nur mit stark verdünnten Lösungen durchgeführt werden, wodurch die Produktivität nachteilig beeinflusst wird.

Die Verwendung von Sauerstoff oder Luft als Oxydationsmittel bringt zahlreiche andere Nachteile mit sich. Die Oxydation verläuft sehr langsam, weshalb auch bei Anwesenheit von Katalysatoren sehr lange Umlaufzeiten erforderlich sind. Die Produktivität wird somit nachteilig beeinflusst. Während der Oxydation muss die Reaktionslösung, damit die Ausbeute durch Bildung von gefärbten Nebenprodukten nicht nachteilig beeinflusst wird, ständig mit Sauerstoff gesättigt sein [vgl. Chimie et Industrie, Genie Chimique 101, 1439–1447 (1969)].

Alle bis jetzt aus der Literatur bekanntgewordenen oxydativen Kondensationen von 4-Nitrotoluol-2-sulfonsäure zu 4,4′-Dinitrostilben-2,2′-disulfonsäure und deren Salze wurden nur unter Verwendung von wässrigen Systemen durchgeführt.

Es ist aber aus der Literatur bekannt, dass Nitro-, Dinitro- und Trinitro-toluole in organischen Lösungsmitteln in Gegenwart von starken Basen und in An- oder Abwesenheit von Katalysatoren durch Sauerstoff (Luft) zu komplexen Mischungen von Produkten, die entsprechende Nitrostilben-Verbindungen enthalten, oxydiert werden können [vgl. C.A. 84, 58.886 n (1976); Acta Chem. Scand. 25, 3509–3516 (1971); J. Org. Chem. 32, 137–46 (1967); Advan. Chem. Ser. 51, 112–71 (1965)].

Diese Oxydationen verlaufen aber mit schlechten Ausbeuten an Nitrostilben-Verbindungen und sind immer mit der Bildung von beträchtlichen Mengen an Nebenprodukten begleitet.

Es wurde nun überraschenderweise gefunden, dass man 4,4′-Dinitrostilben-2,2′-disulfonsäure und deren Salze gegenüber den nächstvergleichbaren Verfahren gemäss C.A. 84, 58.886 n (1976) und Acta Chem. Scand. 25, 3509–3516 (1971) in höheren Ausbeuten und unter Vermeidung der obenerwähnten Nachteile durch Oxydation von 4-Nitrotoluol-2-sulfonsäure herstellen kann, wenn man die Oxydation in einem organischen Lösungsmittel der im Anspruch 1 genannten Art durchführt.

Das erfindungsgemässe Verfahren zur Herstellung von 4,4′-Dinitrostilben-2,2′-disulfonsäure oder deren Salzen der Formel

(1) 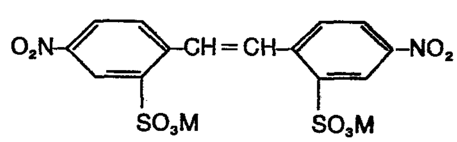

worin M Wasserstoff oder ein Alkalimetallion bedeutet, durch Oxydation von 4-Nitrotoluol-2-sulfonsäure, ist dadurch gekennzeichnet, dass man die Oxydation mit Sauerstoff oder seinen Gemischen mit inerten Gasen oder mit Luft in einem Gemisch aus einem organischen aprotischen dipolaren Lösungsmittel der Formel

(2) 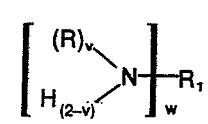

in welcher R eine niedere Alkylgruppe mit 1 bis 4 Kohlenstoffatomen, $R_1$ den Rest einer niedermolekularen Carbonsäure mit 1 bis 4 Kohlenstoffatomen oder den Phosphorsäurerest, w die Basizität der Säure und v die Zahlen 0, 1 oder 2 bedeuten, N-Methylpyrrolidon, Dimethylsulfoxid, Sulfolan, Acetonitril, Tetramethylharnstoff oder deren Gemischen und aus einem aliphatischen niedermolekularen Alkohol mit 1 bis 4 Kohlenstoffatomen und in Gegenwart von mindestens äquivalenten Mengen von stark basischen Alkali- oder Erdalkalimetallverbindungen oder von Gemischen aus solchen Verbindungen durchführt.

Die erfindungsgemässe Oxydation wird in Ab- oder vorzugsweise Anwesenheit von Katalysatoren durchgeführt.

Als Alkalimetallionen M kommen besonders Natrium- und Kaliumionen in Betracht.

Die als Ausgangsmaterial verwendete 4-Nitrotoluol-2-sulfonsäure ist eine bekannte Verbindung, die sehr leicht durch Sulfonierung von 4-Nitrotoluol hergestellt wird.

Bevorzugt sind solche Verbindungen der allgemeinen Formel (2), in denen v = 2 bedeutet. Unter diesen finden insbesondere Dimethylformamid, Hexamethylphosphorsäuretrisamid, Diäthylformamid, Dimethylacetamid und Diäthylacetamid Interesse. Die Verwendung von Gemischen von einer oder mehreren solchen Verbindungen mit aliphatischen niedermolekularen Alkoholen mit 1 bis 4 Kohlenstoffatomen – insbesondere Methanol – hat sich als besonders vorteilhaft erwiesen.

Die eingesetzten Lösungsmittel können, brauchen aber nicht wasserfrei zu sein. Kleine Mengen Wasser, so wie sie in den technischen Lösungsmitteln vorkommen, stören die Oxydation nicht.

Als stark basische Alkali- oder Erdalkalimetallverbindungen kommen besonders Hydroxide, Amide, Hydride, Alkoholate und Sulfide in Betracht.

Als Alkoholate kommen im wesentlichen solche zur Anwendung, die sich von offenkettigen, verzweigten oder cyclischen niederen aliphatischen Alkoholen mit 1 bis 8 Kohlenstoffatomen, vorzugsweise 1 bis 4 Kohlenstoffatomen, ableiten. Diese Alkoholate werden bevorzugt als entsprechende alkoholische Lösungen eingesetzt.

Bevorzugt werden die entsprechenden Natrium- oder Kaliumverbindungen verwendet, von besonderer praktischer Bedeutung sind deren Hydroxide, Amide und Alkoholate.

Die genannten starken Basen werden bevorzugt in wasserfreiem Zustand, entweder für sich oder als Gemisch verwendet. Kleine Mengen Wasser, so wie sie in den technischen starken Basen vorkommen, stören die Oxydation nicht.

Die einzusetzende Basenmenge bewegt sich in weiten Grenzen. Obwohl die Base während der Reaktion nicht verbraucht wird und somit an sich eine katalytische Menge ausreichend wäre, verwendet man sie jedoch vorteilhaft in mindestens äquivalenten Mengen. Ein Vielfaches der äquivalenten Menge wird insbesondere dann verwendet, wenn man bei Temperaturen arbeitet, wo ein Teil der Base durch Reaktion mit dem Lösungsmittel verbraucht wird.

Die optimal zuzusetzende Basenmenge kann jedoch leicht durch Vorversuche festgestellt werden und ist sehr oft durch die Löslichkeit der Base in dem verwendeten Reaktionslösungsmittel begrenzt.

Als Katalysatoren kommen Salze, Oxide oder Hydroxide von Schwermetallverbindungen und/oder schwermetallorganischen Verbindungen wie z.B. solche des Co, Mn, Cr, Ce, Fe, Ni, Cu, Ru, Pd, Pt oder Ir in Betracht (vgl. z.B. Homogeneous Catalysis by Metal Complexes, Vol. I, Chapter 2: Activation of molecular oxygen, page 79, Academic Press New York and London 1974). Von besonderer Bedeutung als Katalysatoren sind jedoch die Salze, Oxide oder Hydroxide des Mangans und/oder die manganorganischen Verbindungen wie z.B. Mangansulfat und/oder Manganacetat.

Anorganische oder organische Brom- und/oder Jod-Verbindungen wie z.B. NaJ, KJ, KBr und Ammoniumbromid können auch mit Vorteil verwendet werden.

Phasentransferkatalysatoren können auch mit Vorteil eingesetzt werden, besonders in solchen Fällen, in welchen die zu verwendenden starken Basen eine ungenügende Löslichkeit im verwendeten Lösungsmittel aufweisen.

Bevorzugt sind Salze der Formel

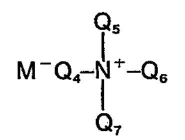 ,

worin

M Fluor, Brom oder Jod, insbesondere Chlor, $Q_4$ Wasserstoff, Alkyl mit 1 bis 18 Kohlenstoffatomen, Cyclohexyl, Benzyl, Phenyl oder Naphthyl und $Q_5$, $Q_6$ und $Q_7$ unabhängig voneinander Wasserstoff oder Alkyl mit 1 bis 18 Kohlenstoffatomen bedeuten, sowie N-Alkyl-Pyridiniumhalogenide mit 1 bis 18 Kohlenstoffatomen im Alkyl, besonders die entsprechenden Chloride.

Beispiele derartiger Salze sind: Ammoniumchlorid, Ammoniumbromid, Methylaminhydrochlorid, Cyclohexylaminhydrochlorid, Anilinhydrochlorid, Dimethylaminhydrochlorid, Di-isobutylaminohydrochlorid, Triäthylaminhydrochlorid, Triäthylaminhydrobromid, Tri-n-octylaminhydrochlorid, Benzyl-dimethylaminhydrochlorid, Tetramethyl-, Tetraäthyl-, Tetra-n-propyl, Tetra-n-butylammoniumchlorid, -bromid und -jodid, Trimethyl-hexadecylammoniumchlorid, Benzyldimethyl-hexadecylammoniumchlorid, Benzyldimethyltetradecylammoniumchlorid, Benzyltrimethyl-, -triäthyl- und tri-n-butylammoniumchlorid, n-Butyl-tri-n-propylammoniumbromid, Octadecyltrimethylammoniumbromid, Phenyltrimethylammoniumbromid oder -chlorid, Hexadecylpyridiniumbromid und -chlorid.

Die Menge des eingesetzten Katalysators kann innerhalb breiter Grenzen variieren. In manchen Fällen genügt es, wenn der Katalysator in Spuren vorliegt. Im allgemeinen wird jedoch der Katalysator bevorzugt in einer Menge von etwa 0,1 bis 15 Gewichtsprozent, bezogen auf die 4-Nitrotoluol-2-sulfonsäure, eingesetzt. Zusätze offenkettiger oder makrocyclischer Polyäther (Kronenäther) sind für den raschen Ablauf von Vorteil. Beispiele solcher Kronenäther sind: 15-Crown-5, 18-Crown-6, Dibenzo-18-crown-6, Dicyclohexyl-18-crown-6, 5,6,14,15-Dibenzo-7,13-diaza-1,4-dioxa-cyclopentadeca-5,14-dien.

Die Reaktionstemperatur ist im allgemeinen nicht kritisch und kann zwischen $-20\,°C$ und dem Siedepunkt des Lösungsmittels oder Lösungsmittelgemisches liegen, vorzugsweise liegt sie jedoch zwischen $-10$ und $50\,°C$, insbesondere zwischen 0 und $25\,°C$.

Als Oxydationsmittel kommen reiner Sauerstoff oder seine Gemische mit inerten Gasen wie z.B. Stickstoff, insbesondere Luft in Frage, wobei die Oxydation bei atmosphärischem Druck oder unter Druck durchgeführt werden kann.

Die erfindungsgemässe Oxydation ist sehr schwach exotherm und verläuft ziemlich rasch auch bei tiefen Temperaturen und ohne Bildung von gefärbten Nebenprodukten.

Das Ausbleiben der gefärbten Nebenprodukte ermöglicht, dass die erfindungsgemäss hergestellte 4,4'-Dinitrostilben-2,2'-disulfonsäure und deren Salze ohne Isolierung zum für die Herstellung von Farbstoffen und optischen Aufhellern wichtigen Zwischenprodukt 4,4'-Diaminostilben-2,2'-disulfonsäure gemäss Anspruch 11 in an sich bekannter Weise weiter reduziert oder zur Herstellung von optischen Aufhellern gemäss Anspruch 13 und von Farbstoffen gemäss Anspruch 12 in an sich bekannter Weise weiter umgesetzt werden kann [vgl. Kirk-Othmer, Encyclopedia of Chemical Technology, 19, 1–14 (1969)].

Die nachfolgenden Beispiele veranschaulichen die Erfindung, ohne sie darauf zu beschränken. Prozente sind, soweit nichts anderes vermerkt, Gewichtsprozente.

Beispiel 1

50 ml Dimethylformamid werden auf $0\,°C$ gekühlt und bei dieser Temperatur unter Rühren und Kühlen mit 108 g einer methanolischen 30%igen Natriummethylat-Lösung verdünnt. Die erhaltene Lösung wird mit 1 g Mangansulfat-Monohydrat versetzt und bei $0\,°C$ durch Einleiten von einem trockenen Luftstrom von 5 l/Std. durch eine in die Lösung eintauchende Fritte während 15 Minuten mit Luft gesättigt.

Zu dieser Lösung wird nun bei 0 bis $5\,°C$ unter Rühren, innerhalb einer Stunde, eine Lösung von 48 g 4-Nitrotoluol-2-sulfonsäure-natriumsalz der Formel

(100) 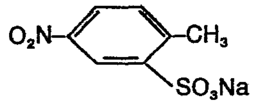

in 150 ml Dimethylformamid zugetropft, während durch die in die Lösung eintauchende Fritte der trockene Luftstrom von 5 l/Std. weitereingeleitet wird. Das während der Zugabe des Natriumsalzes der 4-Nitrotoluol-2-sulfonsäure zu einer dunkelgrünen Suspension gewordene Reaktionsgemisch wird nun 5 weitere Stunden bei 0 bis $5\,°C$ unter Einleiten von 5 l/Std. trockener Luft weitergerührt.

Schon nach etwa 3 Stunden verschwindet die grüne Farbe, und das Reaktionsgemisch wandelt sich durch partielles Ausfallen des gebildeten Reaktionsproduktes in eine gelbe Suspension um, die nach weiteren 2 Stunden mit 108 ml konzentriertem Salzsäure-Wasser (1:1) bei 0 bis $5\,°C$ neutralisiert, unter Vakuum von den Lösungsmitteln befreit und in 200 ml Wasser aufgenommen wird. Das Reaktionsprodukt wird mit 200 ml Sole ausgesalzt, das ausgefallene Produkt abgenutscht, mit 100 ml Sole-Wasser (2:1) gewaschen und bis zum konstanten Gewicht getrocknet. Man erhält 55 g von 4,4'-Dinitrostilben-2,2'-disulfonsäure-dinatriumsalz der Formel

(101) 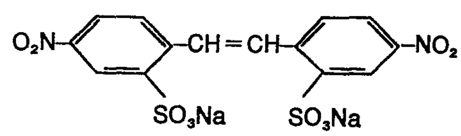

in Form eines gelben kristallinen Pulvers vom Schmelzpunkt über $300\,°C$, das einen NaCl-Gehalt von 16,2% und einen Aktivgehalt (UV-spektralphotometrisch bestimmt) von 94,8% aufweist. Die Ausbeute an 4,4'-Dinitrostilben-2,2'-disulfonsäure-Dinatriumsalz beträgt 92,1% der Theorie.

Ähnliche Resultate werden durch Anwendung von Hexamethylphosphorsäuretrisamid, Diäthylformamid, Diäthylacetamid oder Dimethylacetamid an Stelle von Dimethylformamid erhalten.

Beispiel 2

Das Beispiel 1 wird wiederholt mit dem Unterschied, dass das Reaktionsgemisch nach der Zugabe der Dimethylformamid-Lösung des 4-Nitrotoluol-2-sulfonsäure-natriumsalzes nur 3 Stunden anstatt 5 Stunden bei 0 bis $5\,°C$ unter Einleiten von 5 l/Std. trockener Luft weitergerührt wird.

Man erhält 53,2 g 4,4'-Dinitrostilben-2,2'-disulfonsäure-dinatriumsalz in Form eines gelben kristallinen Pulvers vom Schmelzpunkt über $320\,°C$, das einen NaCl-Gehalt von 16,4% und einen Aktivgehalt (UV-spektralphotometrisch bestimmt) von 94% aufweist.

Die Ausbeute an 4,4'-Dinitrostilben-2,2'-disulfonsäure-dinatriumsalz beträgt 88,2% der Theorie.

Beispiel 3

Das Beispiel 1 wird mit den folgenden Unterschieden wiederholt:

a) An Stelle von 108 g einer methanolischen 30%igen Natriummethylat-Lösung werden 72 g von dieser Lösung verwendet.

b) An Stelle von einem trockenen Luftstrom von 5 l/Std. wird ein trockener Luftstrom von 8 l/Std. verwendet.

c) Das Reaktionsgemisch wird nach der Zugabe der Dimethylformamid-Lösung des 4-Nitrotoluol-2-sulfonsäure-natriumsalzes nur 4 anstatt 5 Stunden bei 0 bis 5 °C unter Einleiten von 8 l/Std. trockener Luft weitergerührt.

Man erhält 51,5 g 4,4'-Dinitrostilben-2,2'-disulfonsäure-dinatriumsalz in Form eines gelben kristallinen Pulvers vom Schmelzpunkt über 340 °C, das einen NaCl-Gehalt von 12,1% und einen Aktivgehalt (UV-spektralphotometrisch bestimmt) von 86,6% aufweist. Die Ausbeute an 4,4'-Dinitrostilben-2,2'-disulfonsäure-dinatriumsalz beträgt 82,7% der Theorie.

Beispiel 4

Das Beispiel 1 wird mit den folgenden Unterschieden wiederholt:

a) An Stelle von 50 ml werden 100 ml Dimethylformamid auf 0 °C gekühlt.

b) An Stelle von 108 g einer methanolischen 30%igen Natriummethylat-Lösung werden 72 g von dieser Lösung verwendet.

c) An Stelle von einem trockenen Luftstrom von 5 l/Std. wird ein trockener Luftstrom von 8 l/Std. verwendet.

d) Das Reaktionsgemisch wird nach der Zugabe der Dimethylformamid-Lösung des 4-Nitrotoluol-2-sulfonsäure-natriumsalzes nur 4 anstatt 5 Stunden bei 0 bis 5 °C unter Einleiten von 8 l/Std. trockener Luft weitergerührt.

Man erhält 46 g 4,4'-Dinitrostilben-2,2'-disulfonsäure-dinatriumsalz in Form eines gelben kristallinen Pulvers vom Schmelzpunkt über 340 °C, das einen NaCl-Gehalt von 11,1% und einen Aktivgehalt (UV-spektralphotometrisch bestimmt) von 98,2% aufweist. Die Ausbeute an 4,4'-Dinitrostilben-2,2'-disulfonsäure-dinatriumsalz beträgt 84,7% der Theorie.

Beispiel 5

Das Beispiel 1 wird mit den folgenden Unterschieden wiederholt:

a) An Stelle von 50 ml werden 100 ml Dimethylformamid auf 0 °C gekühlt.

b) An Stelle von 108 g einer methanolischen 30%igen Natriummethylat-Lösung werden 36 g von dieser Lösung verwendet.

c) An Stelle von einem trockenen Luftstrom von 5 l/Std. wird ein trockener Luftstrom von 8 l/Std. verwendet.

d) Das Reaktionsgemisch wird nach der Zugabe der Dimethylformamid-Lösung des 4-Nitrotoluol-2-sulfonsäure-Natriumsalzes 6 anstatt 5 Stunden bei 0 bis 5 °C unter Einleiten von 8 l/Std. trockener Luft weitergerührt.

Man erhält 35 g 4,4'-Dinitrostilben-2,2'-disulfonsäure-dinatriumsalz in Form eines gelben kristallinen Pulvers vom Schmelzpunkt über 340 °C, das einen NaCl-Gehalt von 93,8% und einen Aktivgehalt (UV-spektralphotometrisch bestimmt) von 96,7% aufweist. Die Ausbeute an 4,4'-Dinitrostilben-2,2'-disulfonsäure-dinatriumsalz beträgt 61,6% der Theorie.

Beispiel 6

(ohne aliphatischen niedermolekularen Alkohol)

27,8 g gepulvertes Natriummethylat (97%ig) werden in 190 ml Dimethylformamid aufgenommen. Die erhaltene Suspension wird mit 1 g Mangansulfat-Monohydrat und 7 g Benzyl-tri-n-butyl-ammoniumbromid versehen und auf 0 °C abgekühlt. Die Suspension wird nun bei 0 bis 5 °C unter Rühren durch Einleiten von einem trockenen Luftstrom von 10 l/Std. bei einem Druck (im Reaktionskolben) von 55 Torr. durch eine in die Suspension eintauchende Fritte während 15 Minuten mit Luft gesättigt.

Zu dieser Suspension wird nun bei 0 bis 5 °C unter Rühren, innerhalb zweier Stunden, eine Lösung von 48 g 4-Nitrotoluol-2-sulfonsäure-natriumsalz in 100 ml Dimethylformamid zugetropft, während durch die in die Suspension eintauchende Fritte der trockene Luftstrom von 10 l/Std. bei einem Druck (im Reaktionskolben) von 55 Torr. weitereingeleitet wird. Das während der Zugabe des Natriumsalzes der 4-Nitrotoluol-2-sulfonsäure zu einer dunkelgrünen Suspension gewordene Reaktionsgemisch wird nun 6 weitere Stunden bei 0 bis 5 °C unter Einleiten von 10 l/Std. trockener Luft bei einem Druck (im Reaktionskolben) von 55 Torr. weitergerührt.

Das dunkelbraune Reaktionsgemisch wird nun mit einer Lösung von 45 ml konzentrierter Salzsäure in 90 ml Wasser bei 0 bis 5 °C neutralisiert, unter Vakuum von Dimethylformamid befreit und in 150 ml Wasser aufgenommen. Das Reaktionsprodukt wird bei Zimmertemperatur abgenutscht, mit 50 ml einer 7,5%igen NaCl-Lösung gewaschen und bis zum konstanten Gewicht getrocknet. Man erhält 47,07 g von 4,4'-Dinitrostilben-2,2'-disulfonsäure-dinatriumsalz in Form eines gelben kristallinen Pulvers vom Schmelzpunkt über 320 °C, das einen NaCl-Gehalt von 1,8%, einen $H_2O$-Gehalt von 1,6% und einen Aktivgehalt (UV-spektralphotometrisch bestimmt) von 45,7% aufweist. Die Ausbeute an 4,4'-Dinitrostilben-2,2'-disulfonsäure-dinatriumsalz beträgt 43,7% der Theorie.

Beispiel 7

37,5 g Kaliumhydroxyd-Pulver (90%ig) werden in 100 ml Methanol heiss gelöst. Die erhaltene klare Lösung wird auf 10 °C abgekühlt, mit 1 g Mangansulfat-monohydrat versehen und mit 100 ml Dimethylformamid verdünnt, wobei das Kaliumhydroxyd als feine Suspension teilweise ausfällt. Das Reaktionsgemisch wird nun auf 0 bis 5 °C abgekühlt, wobei es gleichzeitig durch Einleiten von einem trockenen Luftstrom von 8 l/Std. durch eine in die Lösung eintauchende Fritte während 15 Minuten mit Luft gesättigt wird. Dieser Luftstrom von 8 l/Std. wird während der ganzen Oxydation konstant gehalten.

In dem entstandenen dicken Brei wird nun bei 0 bis 5 °C, unter Rühren, eine Lösung von 48 g 4-Nitrotoluol-2-sulfonsäure-natriumsalz in 100 ml Dimethylformamid innerhalb zwei Stunden zugetropft, wobei das Reaktionsgemisch sofort dunkelgrün gefärbt wird. Das Reaktionsgemisch wird

dann 6 weitere Stunden bei 0 bis 5 °C weitergerührt, wobei das Reaktionsprodukt unter langsamem Verschwinden der dunkelgrünen Farbe – die dunkelgrüne Farbe ist nach etwa 4 Stunden vollständig verschwunden – immer dicker ausfällt. Der Luftstrom wird nun unterbrochen, und der entstandene dunkelgelbe, kristalline Reaktionsbrei wird bei 0 bis 5 °C mit einer Lösung von 52 ml konzentrierter Salzsäure in 90 ml Wasser neutralisiert. Die hellgelbe kristalline Suspension wird unter Vakuum von Lösungsmitteln befreit, in 200 ml Wasser aufgenommen, mit 200 ml einer gesättigten Kaliumchlorid-Lösung ausgesalzt, das ausgefallene Produkt abgenutscht, mit 100 ml einer Lösung von gesättigtem Kaliumchlorid-Wasser (2:1) gewaschen und bis zum konstanten Gewicht getrocknet.

Man erhält 50,01 g 4,4'-Dinitrostilben-2,2'-disulfonsäure-dikaliumsalz in Form eines gelben kristallinen Pulvers vom Schmelzpunkt über 320 °C, das einen KCl-Gehalt von 4,1% und einen Aktivgehalt (UV-spektralphotometrisch bestimmt) von 69,4% aufweist. Die Ausbeute an 4,4'-Dinitrostilben-2,2'-disulfonsäure-dikaliumsalz beträgt 67% der Theorie.

Ähnliche Resultate werden durch Anwendung von Diäthylformamid, Diäthylacetamid oder Hexamethylphosphorsäuretrisamid an Stelle von Dimethylformamid erhalten.

Beispiel 8

24,5 g Natriumhydroxyd-Pulver (98%ig) werden in 90 ml Methanol heiss gelöst. Die erhaltene fast klare Lösung wird auf 10 °C abgekühlt, mit 1 g Mangansulfat-Monohydrat versehen und mit 100 ml Dimethylformamid verdünnt, wobei das Natriumhydroxyd als feine Suspension teilweise ausfällt. Das Reaktionsgemisch wird nun auf 0 bis 5 °C abgekühlt, wobei es gleichzeitig durch Einleiten von einem trockenen Luftstrom von 8 l/Std. durch eine in die Lösung eintauchende Fritte während 15 Minuten mit Luft gesättigt wird. Dieser Luststrom von 8 l/Std. wird dann während der ganzen Oxydation konstant gehalten.

Zu dem enstandenen dicken Brei wird nun bei 0 bis 5 °C, unter Rühren, eine Lösung von 48 g 4-Nitrotoluol-2-sulfonsäure-natriumsalz in 100 ml Dimethylformamid innerhalb zwei Stunden zugetropft, wobei das Reaktionsgemisch sofort dunkelgrün gefärbt wird. Das Reaktionsgemisch wird nun 6 weitere Stunden bei 0 bis 5 °C weitergerührt, wobei das Reaktionsprodukt unter langsamem Verschwinden der dunkelgrünen Farbe – die dunkelgrüne Farbe ist nach etwa 3 Stunden vollständig verschwunden – immer dicker ausfällt.

Der Luftstrom wird nun unterbrochen, und der entstandene dunkel-gelbe, kristalline Reaktionsbrei wird bei 0 bis 5 °C mit 104 ml konzentriertem Salzsäure-Wasser (1:1) neutralisiert. Die erhaltene hellgelbe kristalline Suspension wird unter Vakuum von Lösungsmitteln befreit und der Rückstand in 200 ml Wasser aufgenommen. Das Reaktionsprodukt wird mit 200 ml Sole ausgesalzen, abgenutscht, mit 100 ml Sole-Wasser (2:1) gewaschen und bis zum konstanten Gewicht getrocknet. Man erhält 50,2 g 4,4'-Dinitrostilben-2,2'-disulfonsäure-dinatriumsalz in Form eines hellgelben kristallinen Pulvers vom Schmelzpunkt über 320 °C, das einen NaCl-Gehalt von 9,3% und einen Aktivgehalt (UV-spektralphotometrisch bestimmt) von 99,2% aufweist. Die Ausbeute an 4,4'-Dinitrostilben-2,2'-disulfonsäure-dinatriumsalz beträgt 95,2% der Theorie.

Das IR-Spektrum dieses Produktes ist mit dem IR-Spektrum eines analytisch reinen Musters von 4,4'-Dinitrostilben-2,2'-disulfonsäure-dinatriumsalz deckungsgleich.

Ähnliche Resultate werden durch Anwendung von Hexamethylphosphorsäuretrisamid, Diäthylformamid oder Diäthylacetamid an Stelle von Dimethylformamid erhalten.

Beispiel 9

Das Beispiel 8 wird mit den folgenden Unterschieden wiederholt.

a) Das Natriumhydroxyd-Pulver wird in 100 ml Methanol an Stelle von 90 ml Methanol heiss gelöst.

b) Das Reaktionsgemisch wird nach der Zugabe der Dimethylformamid-Lösung des 4-Nitrotoluol-2-sulfonsäure-natriumsalzes nur 4 anstatt 6 Stunden bei 0 bis 5 °C unter Einleiten von 8 l/Std. trokkener Luft weitergerührt.

Man erhält 50,1 g 4,4'-Dinitrostilben-2,2'-disulfonsäure-dinatriumsalz in Form eines hellgelben kristallinen Pulvers vom Schmelzpunkt über 320 °C, das einen NaCl-Gehalt von 11,1% und einen Aktivgehalt (UV-spektralphotometrisch bestimmt) von 99,0% aufweist. Die Ausbeute an 4,4'-Dinitrostilben-2,2'-disulfonsäure-dinatriumsalz beträgt 92,9% der Theorie.

Beispiel 10

Das Beispiel 8 wird mit den folgenden Unterschieden wiederholt:

a) An Stelle von 24,5 g Natriumhydroxyd-Pulver werden 32 g Natriumhydroxyd-Pulver verwendet.

b) Die Oxydation wird nicht bei 0 bis 5 °C, sondern bei 20 bis 25 °C durchgeführt.

Man erhält 48,0 g 4,4'-Dinitrostilben-2,2'-disulfonsäure-dinatriumsalz in Form eines dunkelgelben kristallinen Pulvers vom Schmelzpunkt über 340 °C, das einen NaCl-Gehalt von 20,9% und einen Aktivgehalt (UV-spektralphotometrisch bestimmt) von 89,4% aufweist. Die Ausbeute an 4,4'-Dinitrostilben-2,2'-disulfonsäure-dinatriumsalz beträgt 71,6% der Theorie.

Beispiel 11

Das Beispiel 8 wird mit den folgenden Unterschieden wiederholt.

a) An Stelle von 24,5 g Natriumhydroxyd-Pulver werden 16 g Natriumhydroxyd-Pulver verwendet.

b) Das Natriumhydroxyd-Pulver wird nicht in 90 ml, sondern nur in 50 ml Methanol heiss gelöst.

c) Das 4-Nitrotoluol-2-sulfonsäure-natriumsalz wird in 150 ml anstatt 100 ml Dimethylformamid gelöst.

Man erhält 48,0 g 4,4'-Dinitrostilben-2,2'-disulfonsäure-dinatriumsalz in Form eines hellgelben kristallinen Pulvers vom Schmelzpunkt über 320 °C, das einen NaCl-Gehalt von 9,3% und einen Aktivgehalt (UV-spektralphotometrisch bestimmt) von 98,0% aufweist. Die Ausbeute an 4,4'-Dinitrostilben-2,2'-disulfonsäure-dinatriumsalz beträgt 90,1% der Theorie.

Beispiel 12

Das Beispiel 8 wird mit den folgenden Unterschieden wiederholt:

a) An Stelle von 24,5 g Natriumhydroxyd-Pulver werden 12,2 g Natriumhydroxyd-Pulver verwendet.

b) Das Natriumhydroxyd-Pulver wird nicht in 90 ml, sondern nur in 50 ml Methanol heiss gelöst.

c) Das Reaktionsgemisch wird nicht mit 100 ml, sondern mit 140 ml Dimethylformamid verdünnt.

d) Die Dimethylformamid-Lösung von 4-Nitrotoluol-2-sulfonsäure-natriumsalz wird nicht innerhalb zweier Stunden, sondern innerhalb einer Stunde zugetropft und das Reaktionsgemisch dann 7 anstatt 6 Stunden weitergerührt.

Man erhält 44,5 g 4,4'-Dinitrostilben-2,2'-disulfonsäure-dinatriumsalz in Form eines gelben kristallinen Pulvers vom Schmelzpunkt über 320 °C, das einen NaCl-Gehalt von 40,6% und einen Aktivgehalt (UV-spektralphotometrisch bestimmt) von 99,7% aufweist. Die Ausbeute an 4,4'-Dinitrostilben-2,2'-disulfonsäure-dinatriumsalz beträgt 83,6% der Theorie.

Beispiel 13

Das Beispiel 8 wird mit den folgenden Unterschieden wiederholt:

a) Das Natriumhydroxyd-Pulver wird nicht in 90, sondern in 100 ml Methanol heiss gelöst.

b) Die Oxydation wird ohne Katalysator durchgeführt.

Man erhält 42,04 g 4,4'-Dinitrostilben-2,2'-disulfonsäure-dinatriumsalz in Form eines gelben kristallinen Pulvers vom Schmelzpunkt über 320 °C, das einen NaCl-Gehalt von 10,2% und einen Aktivgehalt (UV-spektralphotometrisch bestimmt) von 98,5% aufweist. Die Ausbeute an 4,4'-Dinitrostilben-2,2'-disulfonsäure-dinatriumsalz beträgt 78,5% der Theorie.

Beispiel 14

Das Beispiel 8 wird mit dem Unterschied, dass an Stelle von 1 g Mangansulfat-Monohydrat 0,5 g Katalysator verwendet werden, wiederholt. Man erhält 50,63 g 4,4'-Dinitrostilben-2,2'-disulfonsäure-dinatriumsalz in Form eines gelben kristallinen Pulvers vom Schmelzpunkt über 320 °C, das einen NaCl-Gehalt von 11,7% und einen Aktivgehalt (Uv-spektralphotometrisch bestimmt) von 96,9% aufweist. Die Ausbeute an 4,4'-Dinitrostilben-2,2'-disulfonsäure-dinatriumsalz beträgt 91,4% der Theorie.

Beispiel 15

Das Beispiel 8 wird mit den folgenden Unterschieden wiederholt:

a) An Stelle von 24,5 g Natriumhydroxyd-Pulver werden 8,2 g Natriumhydroxyd-Pulver verwendet.

b) Das Natriumhydroxyd-Pulver wird nicht in 90 ml, sondern nur in 50 ml Methanol heiss gelöst.

c) Als Katalysator wird nicht nur 1 g Mangansulfat-Monohydrat, sondern 1 g Mangansulfat-Monohydrat und 1 g Blei (II)-acetat-Trihydrat verwendet.

Man erhält 48,28 g 4,4'-Dinitrostilben-2,2'-disulfonsäure-dinatriumsalz in Form eines gelben kristallinen Pulvers vom Schmelzpunkt über 300 °C, das einen NaCl-Gehalt von 12,3% und einen Aktivgehalt (UV-spektralphotometrisch bestimmt) von 99,3% aufweist. Die Ausbeute an 4,4'-Dinitrostilben-2,2'-disulfonsäure-dinatriumsalz beträgt 88,7% der Theorie.

Beispiel 16

Das Beispiel 8 wird mit dem Unterschied, dass an Stelle von 1 g Mangansulfat-Monohydrat, 1 g Mangan(II)-nitrat-Tetrahydrat als Katalysator verwendet wird, wiederholt.

Man erhält 47,75 g 4,4'-Dinitrostilben-2,2'-disulfonsäure-dinatriumsalz in Form eines gelben kristallinen Pulvers vom Schmelzpunkt über 300 °C, das einen NaCl-Gehalt von 7,3% und einen Aktivgehalt (UV-spektralphotometrisch bestimmt) von 97,4% aufweist. Die Ausbeute an 4,4'-Dinitrostilben-2,2'-disulfonsäure-dinatriumsalz beträgt 91,0% der Theorie.

Ähnliche Resultate werden erhalten, wenn man das Beispiel 8 mit dem Unterschied wiederholt, dass man an Stelle von Mangansulfat-Monohydrat, Mangandioxyd, Mangan(II)-perchlorat-Hexahydrat, Mangan(III)-acetat-Monohydrat, Mangan-(II)-acetylacetonat, Mangan(II)-carbonat, Mangan-(II)-bromid-Tetrahydrat, Mangan(II)-chlorid-Tetrahydrat, Nickelsulfat-Hexahydrat, Nickel(II)-acetat-Tetrahydrat, Nickel(II)-bromid-Trihydrat, Nickelphthalocyanin, Kobalt(II)-sulfat-Heptahydrat, Kobalt(II)-acetat-Tetrahydrat usw. als Katalysator verwendet.

Beispiel 17

20,4 g Natriumhydroxyd-Griess (98%ig) werden in 90 ml Methanol heiss gelöst. Die erhaltene fast klare Lösung wird auf 10 °C abgekühlt, mit 1 g Mangansulfat-Monohydrat versehen und mit 100 ml Dimethylformamid verdünnt, wobei das Natriumhydroxyd als feine Suspension teilweise ausfällt. Das Reaktionsgemisch wird nun auf 0 bis 5 °C abgekühlt, wobei es gleichzeitig durch Einleiten von einem Luftstrom von 10 l/Std. bei einem Überdruck (im Reaktionskolben) von 55 Torr., durch eine in die Lösung eintauchende Fritte, während 15 Minuten mit Luft gesättigt wird. Dieser Luftstrom von 10 l/Std. und der Luftüberdruck im Reaktionskolben von 55 Torr. werden während der ganzen Oxydation konstant behalten.

Zu dem entstandenen dicken Brei wird nun bei 0 bis 5 °C, unter Rühren eine Lösung von 48 g 4-Nitrotoluol-2-sulfonsäure-natriumsalz in 100 ml Dimethylformamid innerhalb einer Stunde zugetropft, wobei das Reaktionsgemisch sofort dunkelgrün gefärbt wird. Das Reaktionsgemisch wird nun 2 Stunden und 45 Minuten bei 0 bis 5 °C weitergerührt, wobei das Reaktionsprodukt unter

langsamem Verschwinden der dunkelgrünen Farbe – die dunkelgrüne Farbe ist nach etwa 2 Stunden und 15 Minuten bei starkem Rühren vollständig verschwunden – immer dicker ausfällt.

Der Luftstrom wird nun unterbrochen, und der entstandene dunkelgelbe, kristalline Reaktionsbrei wird bei 0 bis 10 °C unter Normaldruck mit einer Lösung von 45 ml konzentrierter Salzsäure in 90 ml Wasser neutralisiert. Die erhaltene hellgelbe kristalline Suspension wird unter Vakuum am Rotationsverdampfer zur Trockene eingeengt.

Das trockene Reaktionsgemisch wird in 150 ml Wasser aufgenommen und am Rotationsverdampfer ohne Vakuum bei 90 °C während 15 Minuten weitergerührt. Die heisse kristalline Suspension lässt man auf Zimmertemperatur stets abkühlen, dann wird sie abgenutscht und mit 50 ml einer 7,5gewichtsprozentigen Natriumchlorid-Lösung gewaschen und bei 100 °C unter Vakuum bis zum konstanten Gewicht getrocknet.

Man erhält 48,65 g 4,4′-Dinitrostilben-2,2′-disulfonsäure-dinatriumsalz in Form eines hellgelben kristallinen Pulvers vom Schmelzpunkt über 320 °C, das einen NaCl-Gehalt von 4,2%, einen Wasser-Gehalt von 1,3% und einen Aktivgehalt (UV-spektralphotometrisch bestimmt) von 98,7% aufweist. Die Ausbeute an 4,4′-Dinitrostilben-2,2′-disulfonsäure-dinatriumsalz beträgt 95,6% der Theorie.

Das IR-Spektrum dieses Produktes ist mit dem IR-Spektrum eines analytisch reinen Musters von 4,4′-Dinitrostilben-2,2′-disulfonsäure-dinatriumsalz deckungsgleich.

Beispiel 18

Das Beispiel 17 wird mit dem Unterschied, dass die Oxydation, anstatt bei einem Luftüberdruck von 55 Torr., in einem Glasautoklav bei einem Luftüberdruck von einer Atmosphäre durchgeführt wird, wiederholt.

Man erhält 49,8 g 4,4′-Dinitrostilben-2,2′-disulfonsäure-dinatriumsalz in Form eines hellgelben kristallinen Pulvers vom Schmelzpunkt über 300 °C, das einen NaCl-Gehalt von 12,9%, einen Wassergehalt von 1,3% und einen Aktivgehalt (UV-spektralphotometrisch bestimmt) von 100% aufweist. Die Ausbeute an 4,4′-Dinitrostilben-2,2′-disulfonsäure-dinatriumsalz beträgt 90,3% der Theorie.

Beispiel 19

Das Beispiel 17 wird mit den folgenden Unterschieden wiederholt:

a) Die Oxydation wird nicht mit Luft, sondern mit Sauerstoff durchgeführt.

b) Die Oxydation wird nicht mit Überdruck, sondern bei Atmosphärendruck durchgeführt.

c) Nach Zugabe der Dimethylformamid-Lösung von 4-Nitrotoluol-2-sulfonsäure-natriumsalz wird das Reaktionsgemisch nicht 2 Stunden und 45 Minuten, sondern nur eine Stunde bei 0 bis 5 °C weitergerührt.

Man erhält 45,11 g 4,4′-Dinitrostilben-2,2′-disulfonsäure-dinatriumsalz in Form eines gelben kristallinen Pulvers vom Schmelzpunkt über 320 °C, das einen NaCl-Gehalt von 3,5%, einen Wassergehalt von 1,8% und einen Aktivgehalt (UV-spektralphotometrisch bestimmt) von 97,0% aufweist. Die Ausbeute an 4,4′-Dinitrostilben-2,2′-disulfonsäure-dinatriumsalz beträgt 87,4% der Theorie.

Beispiel 20

Das Beispiel 17 wird mit den folgenden Unterschieden wiederholt:

a) Die Oxydation wird in Gegenwart von 4,4 g des Crown-aethers 15-Crown-5 durchgeführt.

b) Die Oxydation wird ohne Methanol durchgeführt.

Man erhält 45,37 g 4,4′-Dinitrostilben-2,2′-disulfonsäure-dinatriumsalz in Form eines hellgelben kristallinen Pulvers vom Schmelzpunkt über 320 °C, das einen NaCl-Gehalt von 3,9%, einen Wassergehalt von 3,2% und einen Aktivgehalt (UV-spektralphotometrisch bestimmt) von 44,8% aufweist. Die Ausbeute an 4,4′-Dinitrostilben-2,2′-disulfonsäure-dinatriumsalz beträgt 39,8% der Theorie.

Beispiel 21

Das Beispiel 17 wird wiederholt, wobei nach der Oxydation das neutralisierte Reaktionsgemisch zuerst mit 150 ml Wasser verdünnt und dann auf 60 °C erhitzt wird. Die heisse klare Reaktionslösung wird innerhalb 20 Minuten zu einer Aufschwämmung von 100 g gemahlenen Gusseisenspänen und 1 g Natriumacetat in 10 ml 40%iger Essigsäure unter starkem Rühren bei 90 °C zugetropft. Das Reaktionsgemisch wird nun unter starkem Rühren zwei Stunden am Rückfluss (90 °C) gehalten, dann mit einer Lösung von 35 g Natriumcarbonat-Dekahydrat in 50 ml Wasser auf pH 9 gestellt und heiss abgenutscht. Das klare Filtrat wird unter Vakuum am Rotationsverdampfer zur Trockene eingeengt, der trockene Rückstand in 100 ml Wasser aufgenommen und am Rotationsverdampfer ohne Vakuum bei 90 °C zuerst mit 0,5 g Natriumdithionit, dann mit 100 ml Sole versehen und während 15 Minuten weitergerührt. Man lässt die heisse kristalline Suspension auf Zimmertemperatur abkühlen; dann wird sie abgenutscht und mit 50 ml einer Lösung Sole-Wasser (2:1) gewaschen und bei 100 °C unter Vakuum bis zum konstanten Gewicht getrocknet.

Man erhält 44,17 g 4,4′-Diaminostilben-2,2′-disulfonsäure-dinatriumsalz in Form eines blassgelben feinen kristallinen Pulvers vom Schmelzpunkt über 300 °C, das einen NaCl-Gehalt von 14,4%, einen Wassergehalt von 2,0% und einen Aktivgehalt (UV-spektralphotometrisch bestimmt) von 99,3% aufweist. Die Ausbeute an 4,4′-Diaminostilben-2,2′-disulfonsäure-dinatriumsalz beträgt 88,6% der Theorie, bezogen auf das eingesetzte 4-Nitrotoluol-2-sulfonsäure-Natriumsalz.

**Patentansprüche**

1. Verfahren zur Herstellung von 4,4′-Dinitrostilben-2,2′-disulfonsäure und deren Salzen der Formel

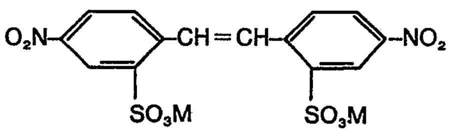

worin M Wasserstoff oder ein Alkalimetallkation bedeutet, durch Oxydation von 4-Nitrotoluol-2-sulfonsäure, dadurch gekennzeichnet, dass man die Oxydation mit Sauerstoff oder seinen Gemischen mit inerten Gasen oder mit Luft in einem Gemisch aus einem organischen aprotischen dipolaren Lösungsmittel der Formel

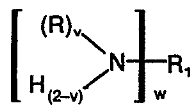

in welcher R eine niedere Alkylgruppe mit 1 bis 4 Kohlenstoffatomen, $R_1$ den Rest einer niedermolekularen Carbonsäure mit 1 bis 4 Kohlenstoffatomen oder den Phosphorsäurerest, w die Basizität der Säure und v die Zahlen 0, 1 oder 2 bedeuten, N-Methylpyrrolidon, Dimethylsulfoxid, Sulfolan, Acetonitril, Tetramethylharnstoff oder deren Gemischen und aus einem aliphatischen niedermolekularen Alkohol mit 1 bis 4 Kohlenstoffatomen und in Gegenwart von mindestens äquivalenten Mengen von stark basischen Alkali- oder Erdalkalimetallverbindungen oder von Gemischen aus solchen Verbindungen durchführt.

2. Verfahren gemäss Anspruch 1, dadurch gekennzeichnet, dass man als aprotisches dipolares Lösungsmittel Dimethylformamid, Hexamethylphosphorsäuretrisamid, Diäthylformamid, Dimethylacetamid oder Diäthylacetamid verwendet.

3. Verfahren gemäss Anspruch 1, dadurch gekennzeichnet, dass man ein Gemisch von Dimethylformamid und/oder Dimethylacetamid und Methanol verwendet.

4. Verfahren gemäss Anspruch 1, dadurch gekennzeichnet, dass man Hydroxide, Amide oder Alkoholate von Alkali- oder Erdalkalimetallen oder Gemische aus solchen Verbindungen als stark basische Verbindungen verwendet.

5. Verfahren gemäss Anspruch 1, dadurch gekennzeichnet, dass man als stark basische Verbindungen Hydroxide oder Alkoholate des Natriums verwendet.

6. Verfahren gemäss Anspruch 1, dadurch gekennzeichnet, dass man die Oxydation bei Temperaturen zwischen $-20\,°C$ und dem Siedepunkt des verwendeten Lösungsmittels durchführt.

7. Verfahren gemäss Anspruch 6, dadurch gekennzeichnet, dass man die Oxydation bei Temperaturen zwischen 0 und 25 °C durchführt.

8. Verfahren gemäss Anspruch 1, dadurch gekennzeichnet, dass man die Oxydation in Anwesenheit eines Katalysators durchführt.

9. Verfahren gemäss Anspruch 8, dadurch gekennzeichnet, dass man als Katalysatoren Salze, Oxide oder Hydroxide von Schwermetall-Verbindungen und/oder schwermetallorganische Verbindungen verwendet.

10. Verfahren gemäss Anspruch 1, dadurch gekennzeichnet, dass man die Oxydation unter Druck durchführt.

11. Verfahren gemäss Anspruch 1, dadurch gekennzeichnet, dass die erhaltene 4,4′-Dinitrostilben-2,2′-disulfonsäure und deren Salze ohne Isolierung noch zu 4,4′-Diamino- oder (4-Amino-4′-nitro)-stilben-2,2′-disulfonsäure und deren Salzen reduziert werden.

12. Verfahren zur Herstellung von eine oder mehrere Stilben-(4,4′)-diyl-2,2′-disulfonsäuregruppen enthaltenden Farbstoffen, dadurch gekennzeichnet, dass man 4-Nitrotoluol-2-sulfonsäure gemäss Ansprüchen 1 bis 10 zur 4,4′-Dinitrostilben-2,2′-disulfonsäure und deren Salzen oxydiert und diese, ohne Isolierung, zu besagten Farbstoffen in an sich bekannter Weise weiterverarbeitet.

13. Verfahren zur Herstellung von eine Stilben-(4,4′)-diyl-2,2′-disulfonsäuregruppe enthaltenden optischen Aufhellern, dadurch gekennzeichnet, dass man 4-Nitrotoluol-2-sulfonsäure gemäss Ansprüchen 1 bis 10 zur 4,4′-Dinitrostilben-2,2′-disulfonsäure und deren Salzen oxydiert und diese, ohne Isolierung, zu besagten optischen Aufhellern in an sich bekannter Weise weiterverarbeitet.

**Claims**

1. A process for the preparation of 4,4′-dinitrostilbene-2,2′-disulfonic acid, or a salt thereof, of the formula

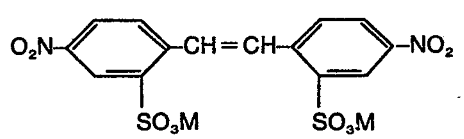

in which M is hydrogen or an alkali metal cation, by oxidation of 4-nitrotoluene-2-sulfonic acid, which comprises carrying out the oxidation with oxygen or a mixture thereof with an inert gas or with air, in a mixture of an organic aprotic dipolar solvent of the formula

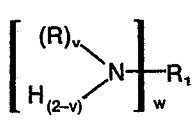

in which R is a lower alkyl group having 1 to 4 carbon atoms, $R_1$ is the radical of a low-molecular carboxylic acid having 1 to 4 carbon atoms, or the phosphoric acid radical, w is the basicity of the acid and v is 0, 1 or 2, or also N-methylpyrrolidone, dimethylsulfoxide, sulfolane, acetonitrile, tetramethylurea or a mixture thereof, and of an aliphatic low molecular alcohol containing 1 to 4 carbon atoms and in the presence of at least an equivalent amount of strongly basic alkali metal compounds or alkaline earth metal compounds or of mixtures thereof.

2. A process according to claim 1, wherein the aprotic dipolar solvent used is dimethylformamide, hexamethylphosphoric acid triamide, diethylformamide, dimethylacetamide or diethylacetamide.

3. A process according to claim 1, wherein a mixture of dimethylformamide and/or dimethylacetamide and methanol is used.

4. A process according to claim 1, wherein hydroxides, amides or alcoholates of alkali metals or alkaline earth metals, or mixtures thereof, are used as strongly basic compounds.

5. A process according to claim 1, wherein the strongly basic compounds are hydroxides or alcoholates of sodium.

6. A process according to claim 1, wherein the oxidation is carried out in the temperature range from −20 °C to the boiling point of the solvent used.

7. A process according to claim 6, wherein the oxidation is carried out in the temperature range from 0 to 25 °C.

8. A process according to claim 1, wherein the oxidation is carried out in the presence of a catalyst.

9. A process according to claim 8, wherein the catalyst is a salt, oxide or hydroxide of a heavy metal compound and/or a heavy metal-organic compound.

10. A process according to claim 1, wherein the oxidation is carried out under pressure.

11. A process according to claim 1, wherein the resultant 4,4′-dinitrostilbene-2,2′-disulfonic acid, or salt thereof, is reduced, without isolation, to 4,4′-diamino- or (4-amino-4′-nitro)-stilbene-2,2′-disulfonic acid, or a salt thereof.

12. A process for the preparation of a dye containing one or more stilbene-(4,4′)-diyl-2,2′-disulfonic acid groups, which comprises oxidising 4-nitrotoluene-2-sulfonic acid according to any one of claims 1 to 10, in organic solvents, to give 4,4′-dinitrostilbene-2,2′-disulfonic acid, or a salt thereof, and further processing this acid or salt, without isolation, in a manner known per se to give the said dye.

13. A process for the preparation of a fluorescent brightening agent containing a stilbene-(4,4′)-diyl-2,2′-disulfonic acid group, which comprises oxidising 4-nitrotoluene-2-sulfonic acid according to any one of claims 1 to 10, to give 4,4′-dinitrostilbene-2,2′-disulfonic acid, or a salt thereof, and further processing this acid or salt, without isolation, in a manner known per se to give the said fluorescent brightening agent.

**Revendications**

1. Procédé pour la préparation de l'acide 4,4′-dinitrostilbène-2,2′-disulfonique et de ses sels de formule:

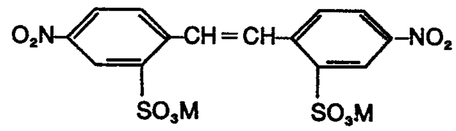

dans laquelle M est de l'hydrogène ou un cation de métal alcalin, par oxydation de l'acide 4-nitrotoluène-2-sulfonique, caractérisé par le fait qu'on effectue l'oxydation avec de l'oxygène ou avec ses mélanges avec des gaz inertes ou avec de l'air, dans un mélange constitué par un solvant organique, dipolaire, aprotique, de formule:

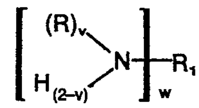

dans laquelle R est un groupe alkyle inférieur ayant 1 à 4 atomes de carbone; $R_1$ est le reste d'un acide carboxylique à bas poids moléculaire ayant 1 à 4 atomes de carbone, ou le reste d'acide phosphorique; w est la basicité de l'acide, et v est les nombres 0, 1 ou 2, la N-méthylpyrrolidone, le diméthylsulfoxyde, le sulfolane, l'acétonitrile, la tétraméthylurée ou leurs mélanges et par un alcool aliphatique à bas poids moléculaire, ayant 1 à 4 atomes de carbone, et en présence de quantités au moins équivalentes de composés alcalins ou alcalino-terreux fortement basiques ou des mélanges de ces composés.

2. Procédé selon la revendication 1, caractérisé par le fait qu'on utilise comme solvant dipolaire aprotique, le diméthylformamide, l'hexaméthylphosphoryltrisamide, le diéthylformamide, le diméthylacétamide ou le diéthylacétamide.

3. Procédé selon la revendication 1, caractérisé par le fait qu'on utilise un mélange de diméthylformamide et/ou de diméthylacétamide et de méthanol.

4. Procédé selon la revendication 1, caractérisé par le fait qu'on utilise des hydroxydes, des amides ou des alcoolates, de métaux alcalins ou alcalino-terreux ou des mélanges de ces composés comme composés fortement basiques.

5. Procédé selon la revendication 1, caractérisé par le fait qu'on utilise comme composés fortement basiques l'hydroxyde ou des alcoolates de sodium.

6. Procédé selon la revendication 1, caractérisé par le fait qu'on effectue l'oxydation à des températures comprises entre −20 °C et le point d'ébullition du solvant utilisé.

7. Procédé selon la revendication 6, caractérisé par le fait qu'on effectue l'oxydation à des températures comprises entre 0 et 25 °C.

8. Procédé selon la revendication 1, caractérisé par le fait qu'on effectue l'oxydation en présence d'un catalyseur.

9. Procédé selon la revendication 8, caractérisé par le fait qu'on utilise comme catalyseurs: des sels, des oxydes ou des hydroxydes de composés métalliques lourds et/ou des composés organiques métalliques lourds.

10. Procédé selon la revendication 1, caractérisé par le fait qu'on effectue l'oxydation sous pression.

11. Procédé selon la revendication 1, caractérisé par le fait que l'acide 4,4′-dinitrostilbène-2,2′-disulfonique obtenu et ses sels sont encore réduits, sans isolation, en acides 4,4′-diamino- ou (4-amino-4′-nitro)-stilbène-2,2′-disulfoniques et leurs sels.

12. Procédé pour la préparation de colorants contenant un ou plusieurs groupes acide stilbène-(4,4′)-diyl-2,2′-disulfonique, caractérisé par le fait qu'on oxyde l'acide 4-nitrotoluène-2-sulfonique, selon les revendications 1 à 10, en acide 4,4′-dinitrostilbène-2,2′-disulfonique et en ses sels et qu'on transforme ces produits, sans isolation, en colorants en question d'une façon connue.

13. Procédé pour la préparation d'azurants optiques contenant un groupe acide stilbène-(4,4′)-diyl-2,2′-disulfonique, caractérisé par le fait qu'on oxyde l'acide 4-nitrotoluène-2-sulfonique, selon les revendications 1 à 10, en acide 4,4′-dinitrostilbène-2,2′-disulfonique et en ses sels et qu'on transforme ces produits, sans isolation, en azurants optiques en question de façon connue.